# EUROPÄISCHE PATENTSCHRIFT

(11) **EP 0 865 423 B1**
(45) Veröffentlichungstag und Bekanntmachung des Hinweises auf die Patenterteilung: **23.04.2003**
(21) Anmeldenummer: 96934559.4
(22) Anmeldetag: 07.10.1996
(51) Int. Cl.: C07C 251/24, A01N 37/50

(54) **IMINOESSIGSÄUREAMIDE UND IHRE VERWENDUNG ALS SCHÄDLINGSBEKÄMPFUNGSMITTEL**
IMINOACETIC ACID AMIDES AND THEIR USE AS PEST CONTROL AGENTS
AMIDES D'ACIDE IMINOACETIQUE ET LEUR UTILISATION EN TANT QUE PESTICIDES

(30) Priorität: 18.10.1995 DE 19538789
(43) Veröffentlichungstag der Anmeldung: 23.09.1998
(73) Patentinhaber: Bayer CropScience AG, 40789 Monheim (DE)
(72) Erfinder: SEITZ, Thomas, D-40764 Langenfeld (DE); STENZEL, Klaus, D-40595 Düsseldorf (DE)
(86) Internationale Anmeldenummer: EP9604345
(87) Internationale Veröffentlichungsnummer: WO97014673

(56) Entgegenhaltungen:
- EP-A- 0 602 514
- CHEMICAL ABSTRACTS, vol. 65, no. 1, 4.Juli 1966 Columbus, Ohio, US; ATSUO KANAI: "Studies on asymmetric hydrogenation. VI Asymmetric catalytic hydrogenation of of the S(-)-alpha-phenylethylamine salt and amide of 2-[S(-)-alphaphenylethylimino]-2-phenylace tic acid" Spalte 16835; XP002023285 & NIPPON KAGAKU ZASSHI, Bd. 87, Nr. 2, 1966, Seite 18
- INDIAN JOURNAL OF CHEMISTRY, Bd. 21B, 1982, Seiten 348-351, XP000614358 A.S.HAMMAM ET AL.: "Synthesis and biological activities of some new peptide substituted carbazoquinones"
- 'The Pesticide Manual, 10th Ed.', 1994 siehe Seite 1137

## Beschreibung

Die Erfindung betrifft neue Iminoessigsäureamide, ein Verfahren zu ihrer Herstellung und ihre Verwendung als Schädlingsbekämpfungsmittel.

Iminoessigsäureamide wurden bereits beschreiben, die Verwendung als Schädlingsbekämpfungsmittel ist nicht beschrieben (vgl. Hamman, Indian Journal of Chemistry 1982, 348-351).

Es wurden neue Verbindungen der allgemeinen Formel (I) gefunden, 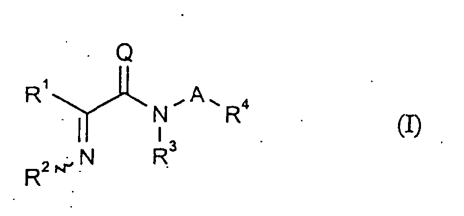 in welcher
- A: für eine Einfachbindung oder für gegebenenfalls einfach oder mehrfach, gleich oder verschieden durch Fluor, Chlor, Cyano, Methoxy substituiertes Methylen, 1,2-Ethylen 1,1-, 1,2-, 1,3- oder 2,2-Propylen, 1,1-, 1,2-, 1,3-, 1,4-, 2,2- oder 2,3-Butylen oder 1,1-, 1,2- oder 1,3-(2-Methyl-propylen) steht,
- Q: für Sauerstoff oder Schwefel steht,
- R¹: für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy,
Ethylsulfonyloxy, Hydroximinomethyl, Hydroximinoethyl, Methoximinomethyl, Ethoximinomethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy;
Cyclopropyl, Cyclobutyl, Cyclopentyl oder Cyclohexyl;
gegebenenfalls durch die oben genannten Substituenten substituiertes Phenyl, Benzyl, Phenoxy, Benzyloxy,
- R²: für Methyl, Ethyl, n- oder i-Propyl, n-, i-, s- oder t-Butyl oder Propargyl oder Allyl steht,
oder für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzahlung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, n-Pentyl, n-Hexyl, n-Heptyl, Methoxy, Ethoxy. n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl; jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetrarnethylen (Butan-1,4-diyl), Methylendioxy oder Ethylendioxy,
oder für die oben genannten Substituenten substituiertes Benzyl steht,
- R³: für Wasserstoff oder für Methyl oder Ethyl steht,
- R⁴: für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylanino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl, jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

Besonders bevorzugt sind Verbindungen der Formel (I), in welcher
- A: für eine Einfachbindung oder für gegebenenfalls einfach oder mehrfach, gleich oder verschieden durch Fluor, Chlor, Cyano, Methoxy substituiertes Methylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
- Q: für Sauerstoff oder Schwefel steht,
- R¹: für jeweils gegebenenfalls einfach bis sechsfach substituiertes Cyclobutyl, Cyclopentyl oder Cyclohexyl, wobei als Substituenten die unten genannten bevorzugt sind;
für jeweils gegebenenfalls einfach bis dreifach substituiertes Phenyl, Naphthyl, Furyl, Benzofuranyl, Thienyl, Benzothienyl, Pyridyl, Chinolyl, Tetrahydrofuryl oder Perhydropyranyl steht, wobei die möglichen Substituenten vorzugsweise aus der nachstehenden Aufzählung ausgewählt sind:
Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl; jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy; Cyclopropyl, Cyclopentyl, Cyclohexyl; gegebenenfalls durch die oben genannten Substituenten substituierter Phenyl, Phenoxy, Benzyloxy;

- R²: für Methyl, Ethyl, n-, i-Propyl, n-, i-, s- oder t-Butyl, Allyl
oder für jeweils gegebenenfalls einfach bis sechsfach substituiertes Cyclobutyl, Cyclopentyl oder Cyclohexyl, wobei als Substituenten die unten genannten bevorzugt sind;
oder für jeweils gegebenenfalls einfach bis dreifach substituiertes Phenyl, Naphthyl, Furyl, Benzofuranyl, Thienyl, Benzothienyl, Pyridyl, Chinolyl, Tetrahydrofuryl oder Perhydropyranyl steht, wobei die möglichen Substituenten vorzugsweise aus der nachstehenden Aufzählung ausgewählt sind:
Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
oder für die oben genannten Substituenten substituiertes Benzyl steht,
- R³: für Wasserstoff oder für Methyl steht,
- R⁴: für jeweils gegebenenfalls einfach bis sechsfach substituiertes Cyclopropyl, Cyclobutyl, Cyclopentyl oder Cyclohexyl steht, wobei als Substituenten die unten genannten bevorzugt sind; für jeweils gegebenenfalls einfach bis dreifach substituiertes Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

Eine ganz besonders bevorzugte Gruppe erfindungsgemäßer Verbindungen sind diejenigen Verbindungen der Formel (I), in welcher
- A: für eine Einfachbindung oder für Methylen, 1,2-Ethylen, 1,1-Propylen, 1,2-Propylen, 1,3-Propylen oder 2,2-Propylen steht,
- Q: für Sauerstoff steht,
- R¹: für gegebenenfalls durch Brom, Chlor, Fluor, Nitro, Methylsulfonyl, Phenyl, Phenyloxy, Benzyloxy, Cyclopropyl, Cyclohexyl, Methyl, Ethyl, n- oder i-Propyl, n-, i-, s- oder t-Butyl, n-Pentyl, n-Hexyl, n-Heptyl, Methoxy, Ethoxy, Trifluormethyl, Trifluormethoxy und/oder Methylthio einfach oder zweifach substituiertes Phenyl, Thienyl oder Furanyl steht oder für jeweils gegebenenfalls durch Fluor substituiertes 3,4-Methylen- und Ethylendioxo, Propan-1,3-diyl und Butan-1,4-diyl substituiertes Phenyl steht oder für Naphthyl, Benzofuranyl oder Benzothienyl steht,
- R²: für Methyl oder Ethyl, für n- oder i-Propyl, für n-, i-, s- oder t-Butyl, Allyl, Cyclopentyl, Cyclohexyl oder für gegebenenfalls durch Chlor, Methyl oder Methoxy substituiertes Phenyl oder Benzyl steht,
- R³: für Wasserstoff oder Methyl steht,
- R⁴: für Cyclohexyl oder gegebenenfalls einfach bis dreifach substituiertes Phenyl, Thienyl, Furyl, Benzofuryl, Benzothienyl, Pyridyl, Pyrimidinyl, Naphthyl, Chinolyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl, jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy steht.

Von ganz besonderem Interesse sind Verbindungen der Formel (I), in welcher der Rest R¹ für unsubstituiertes oder in 3- und/oder 4-Stellung substituiertes Phenyl, oder für unsubstituiertes oder in 4- und/oder 5-Stellung substituiertes Furanyl oder Thienyl steht, wobei als Substituenten die oben genannten und insbesondere Chlor, Brom, Fluor, Nitro, Methylsulfonyl, Phenyl, Phenoxy, Benzyloxy, Cyclopropyl, Cyclohexyl, Methyl, Ethyl, n-, i-Propyl, n-, i-, s-, t-Butyl, n-Pentyl, n-Hexyl, n-Heptyl, Methoxy, Ethoxy, Methylthio, Trifluormethyl und Trifluormethoxy ausgewählt sind oder jeweils gegebenenfalls durch Fluor substituiertes 3,4-Methylen- und Ethylendioxo, Propan-1,3-diyl und Butan-1,4-diyl substituiertes Phenyl oder in 2-Stellung substituiertes Naphthyl, Benzofuranyl oder Benzothienyl bedeuten.

Insbesondere sind auch Verbindungen der Formel (I) bevorzugt, in denen R⁴ für in 3-und 4-Stellung durch Methoxy substituiertes Phenyl steht.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Bevorzugte Einzelverbindungen können den folgenden Tabellen entnommen werden:

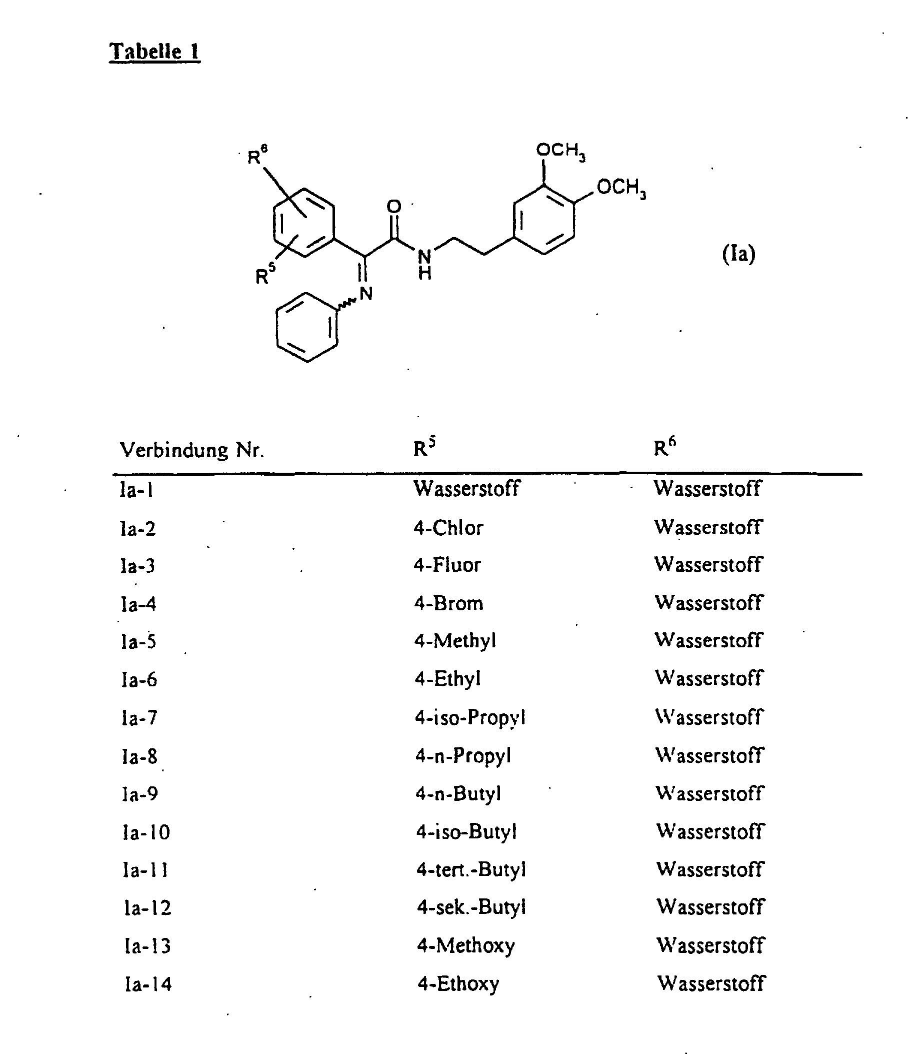

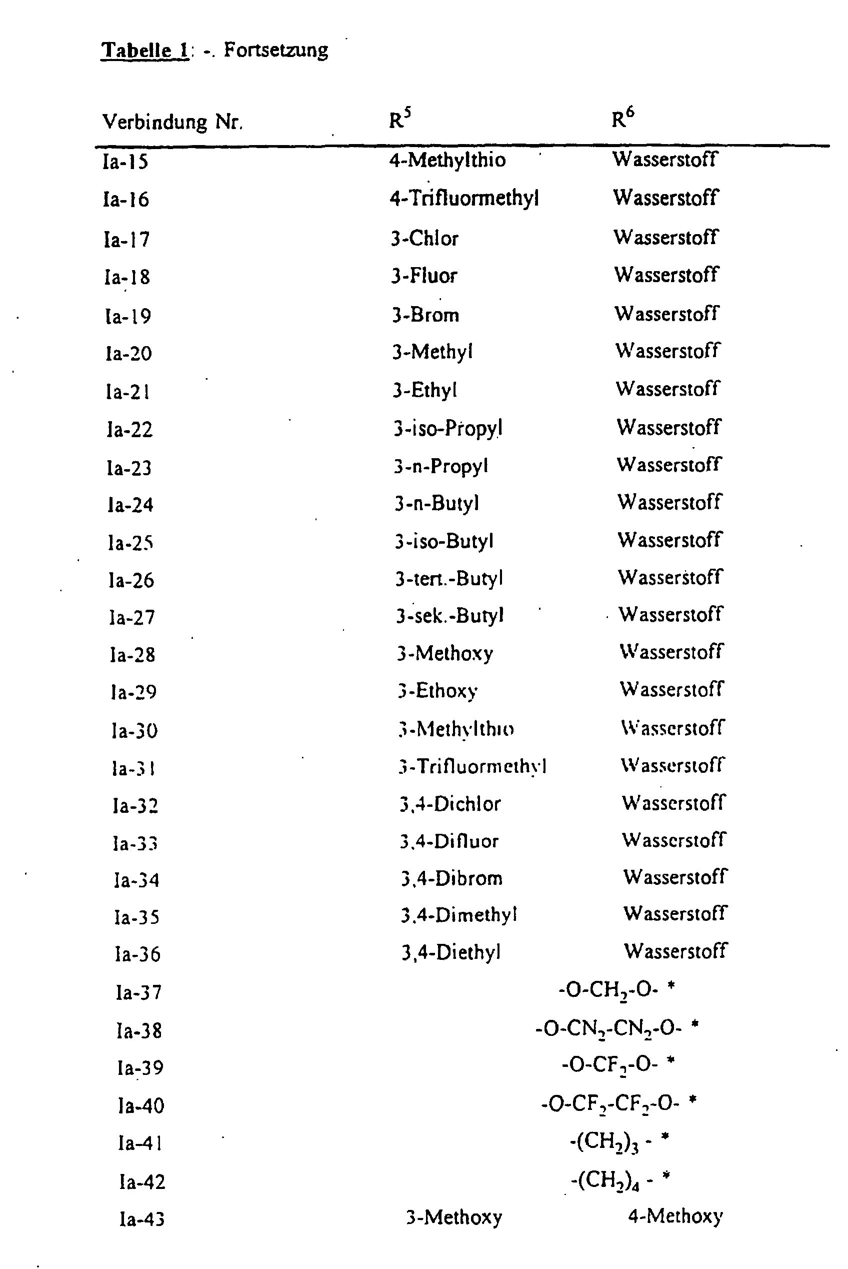

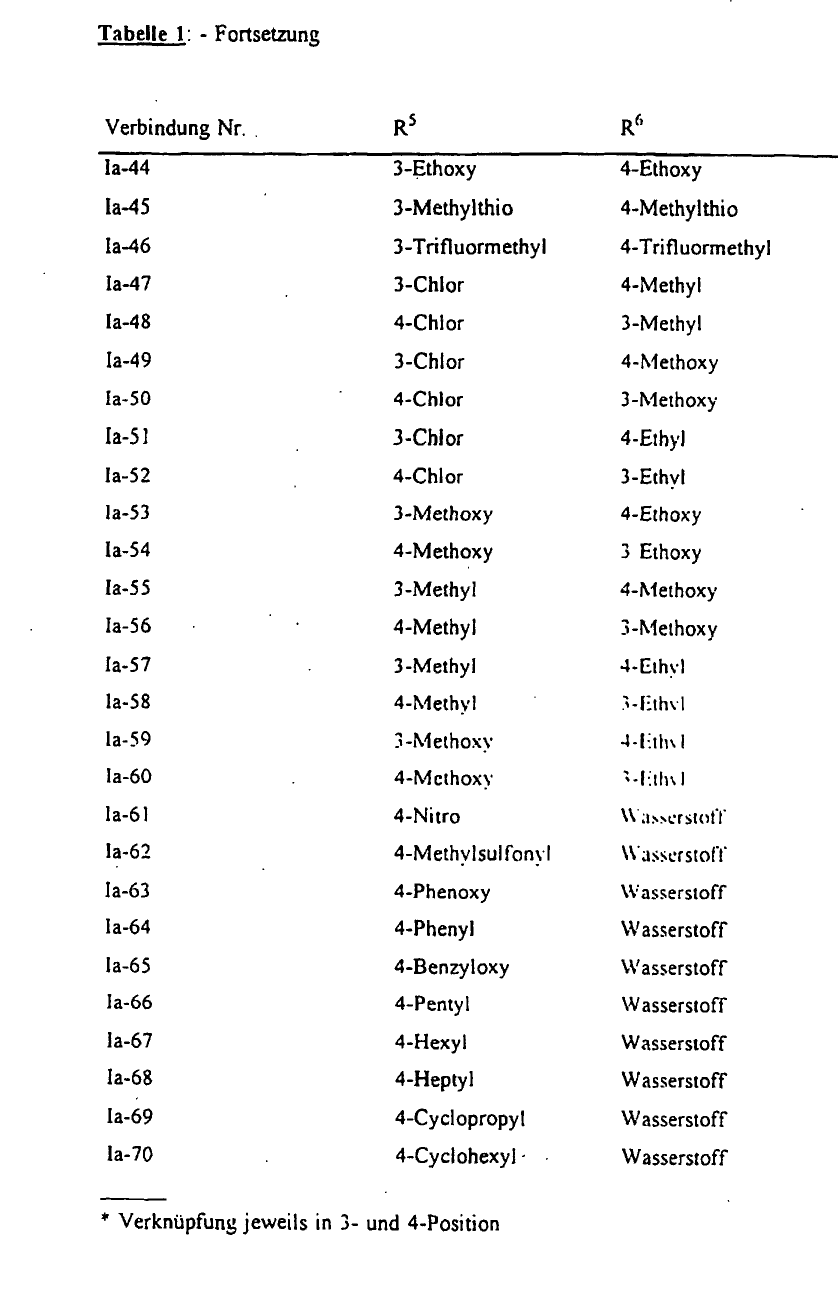

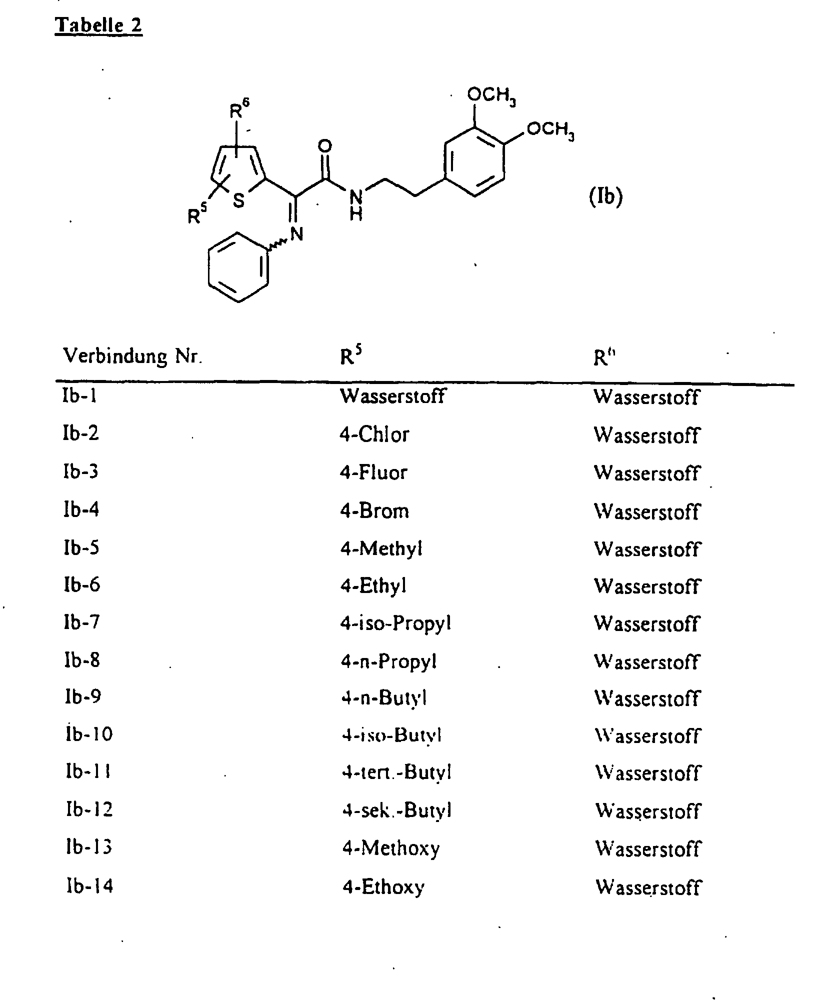

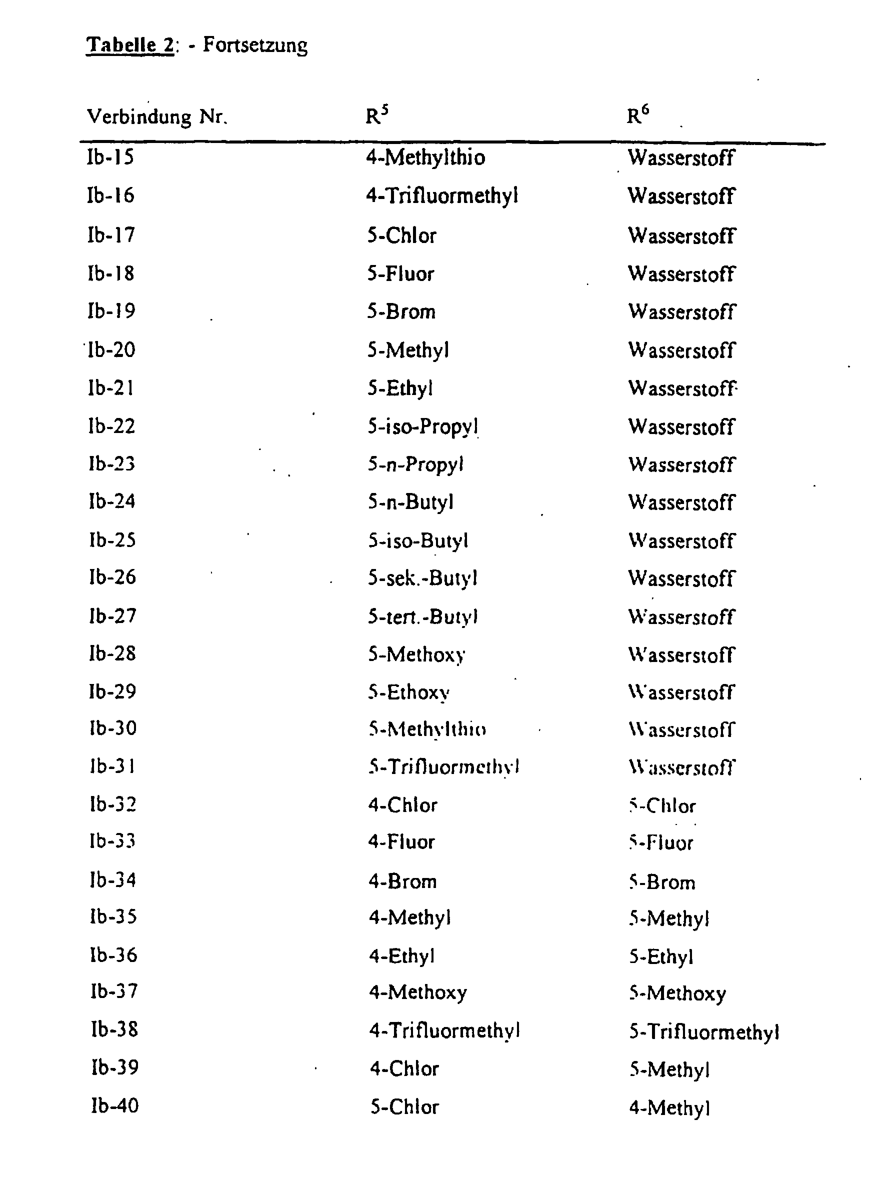

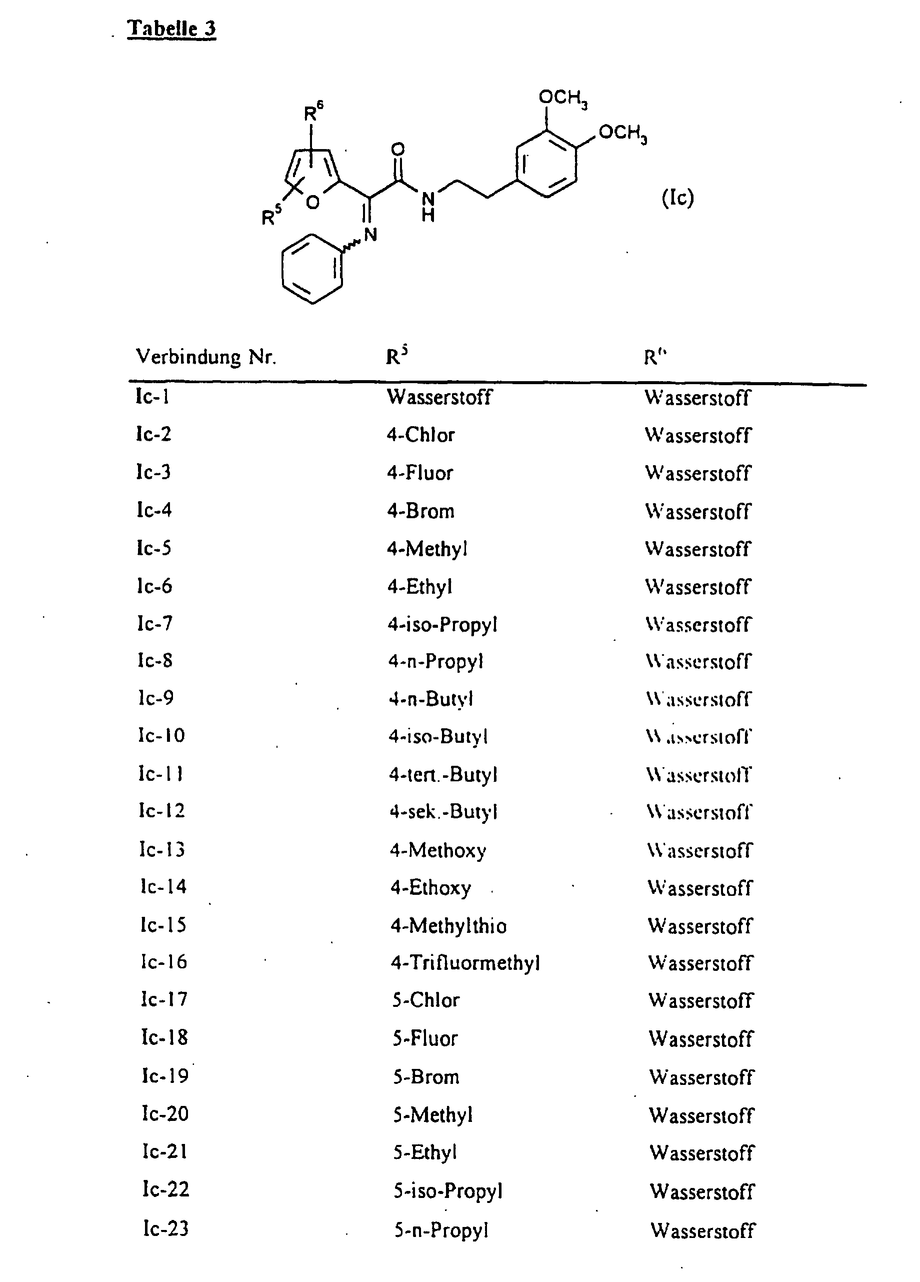

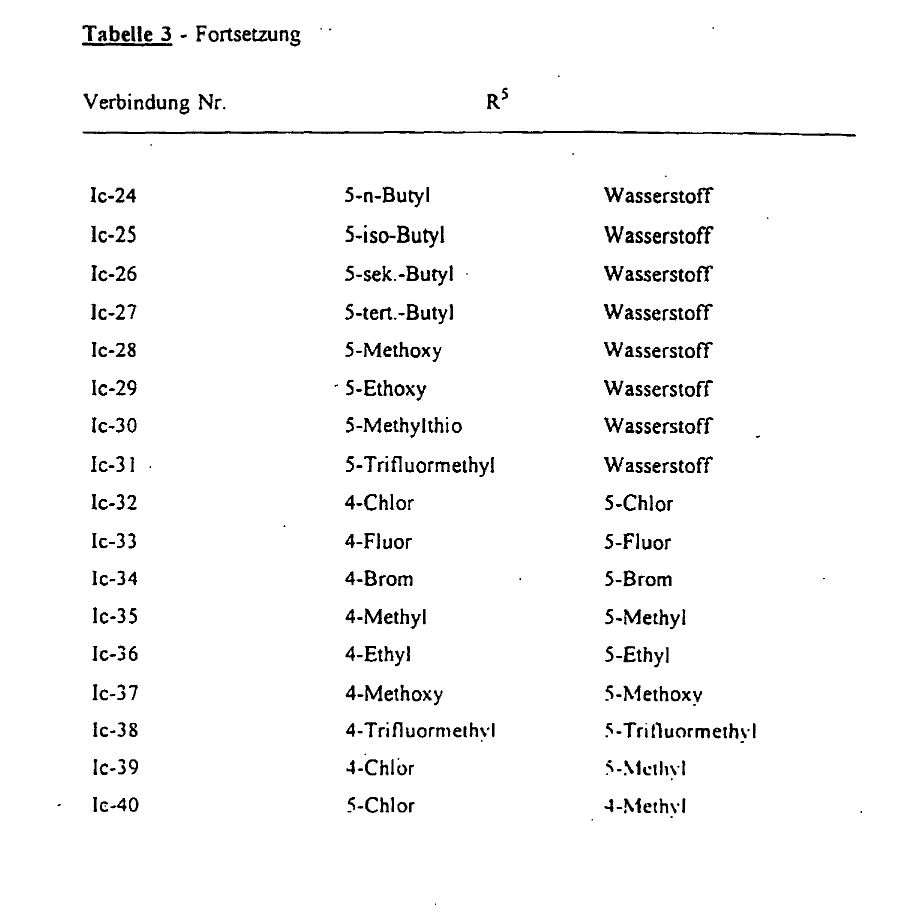

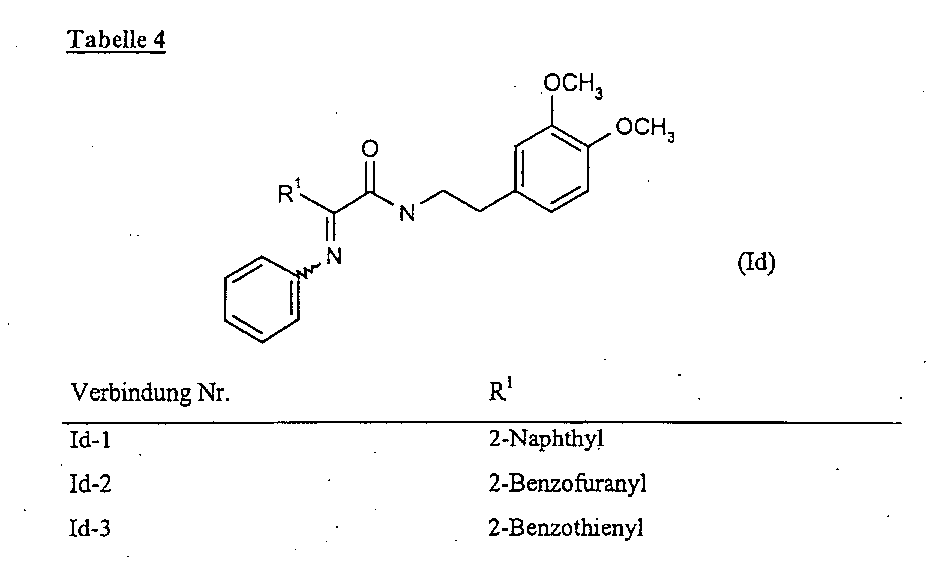

### Tabelle 5

Verbindungen Ia-1 bis Id-3 entsprechend den Formeln Ia, Ib, Ic und Id, wobei an Stelle der 3,4-Dimethoxyphenyl-Gruppe (im allgemeinen bezeichnet als R⁴) einer der folgenden dreifach substituierten Phenylreste steht:Substituenten: 3,4,5-Trimethoxy; 3,4,5-Trichlor; 3,4,5-Trimethyl.

Weiter wurde gefunden, dass man die neuen Iminoessigsäureamide der allgemeinen Formel (I) erhält, wenn man Carbonsäurederivate der allgemeinen Formel (II) 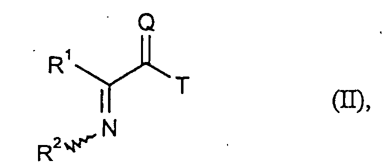 in welcher
- R¹, R² und Q: die oben angegebene Bedeutung haben und
- T: für Hydroxy, Halogen oder Alkoxy steht,
mit einem Amin der allgemeinen Formel (III), 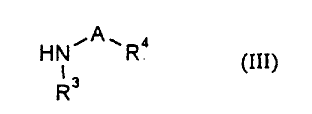 in welcher
R³, R⁴ und A die oben angegebenen Bedeutungen haben,
- oder mit einem Hydrogenhalogenid hiervon -
gegebenenfalls in Gegenwart eines Säureakzeptors, gegebenenfalls in Gegenwart eines Kondensationsmittels, gegebenenfalls in Gegenwart eines Katalysators und gegebenenfalls in Gegenwart eines Verdünnungsmittels umsetzt.

Die zur Durchführung des erfindungsgemäßen Verfahrens als Ausgangsstoffe benötigten Carbonsäurederivate sind durch die Formel (II) allgemein definiert. In dieser Formel (II) haben (Q, R¹ und R²) vorzugsweise insbesondere diejenige Bedeutungen, die bereits im Zusammenhang mit der Beschreibung der erfindungsgemäßen Verbindungen der Formel (I) als bevorzugt bzw. als insbesondere bevorzugt für (Q, R¹ und R²) angegeben wurden. T steht vorzugsweise für Alkoxy mit 1 bis 4 Kohlenstoffatomen, insbesondere fur Methoxy oder Ethoxy, für Hydroxy oder Chlor

Die Ausgangsstoffe der Formel (II) sind bekannt und/oder können nach an sich bekannten Verfahren hergestellt werden (vgl. z B Tetrahedron 1971, 3431-6).

Die weiter als Ausgangsstoffe zu verwendeten Amine sind durch die Formel (III) allgemein definiert. In dieser Formel (II) haben R³, R⁴ und A vorzugsweise insbesondere diejenige Bedeutungen, die bereits im Zusammenhang mit der Beschreibung der erfindungsgemäßen Verbindungen der Formel (I) als bevorzugt bzw. als insbesondere bevorzugt für R³, R⁴ und A angegeben wurden.

Die Ausgangsstoffe der Formel (III) sind bekannte organische Synthesechemikalien und/oder können nach an sich bekannten Verfahren hergestellt werden.

Das erfindungsgemäße Verfahren wird gegebenenfalls in Gegenwart eines geeigneten Säureakzeptors durchgeführt. Als solche kommen alle üblichen anorganischen oder organischen Basen infrage. Hierzu gehören beispielsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N,N-Dimethylaminopyridin, Diazabicyclooctan (DABCO), Diazabicyclononen (DBN) oder Diazabicycloundecen (DBU).

Das erfindungsgemäße Verfahren wird gegebenenfalls in Gegenwart eines geeigneten Kondensationsmittels durchgeführt. Als solche kommen alle üblicherweise für derartige Amidierungsreaktionen verwendbaren Kondensationsmittel infrage. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester oder Methansulfonylchlorid; Carbodiimide. wie N,N'-Dicyclohexylcarbodiimid (DCC) oder andere übliche Kondensalionsmittel, wie Phosphorpentoxid, Polyphosphorsäure, N,N'-Carbonyldiimidazol, 2-Ethoxy-N-ethoxycarbonyl-1,2-dihydrochinolin (EEDQ) oder Triphenylphosphin Tetrachlorkohlenstoff.

Das erfindungsgemäße Verfahren wird gegebenenfalls in Gegenwart eines Verdunnungsmittels durchgeführt. Als Verdünnungsmittel zur Durchführung des erfindungsgemäßen Verfahrens kommen Wasser und organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff, Ether, wie Diethylether, Diisopropylether, Dioxan, Tetrahydrofuran oder Ethylenglykol-dimethyl- oder -diethylether, Ketone, wie Aceton, Butanon oder Methyl-isobutyl-keton, Nitrile, wie Acetonitril, Propionitril oder Benzonitril, Amide, wie N,N-Dimethylformamid, N,N-Dimethylacetamid, N-Methylformanilid, N-Methylpyrrolidon oder Hexamethylphosphorsäuretriamid, Ester wie Essigsäuremethylester oder Essigsäureethylester, Sulfoxide, wie Dimethylsulfoxid, Alkohole, wie Methanol, Ethanol, n- oder i-Propanol, Ethylenglykol, Ethylenglykolmonomethylether, Ethylenglykolmonoethylether, Diethylenglykolmonomethylether, Diethylenglykolmonoethylether, deren Gemische mit Wasser oder reines Wasser.

Das erfindungsgemäße Verfahren wird gegebenenfalls in Gegenwart eines Katalysators durchgeführt. Beispielsweise genannt seien 4-Dimethylaminopyridin, 1-Hydroxy-benzotriazol oder Dimethylformamid.

Die Reaktionstemperaturen können bei der Durchführung des erfindungsgemäßen Verfahrens in einem größeren Bereich variiert werden. Im allgemeinen arbeitet man bei Temperaturen zwischen -20°C und +200°C, vorzugsweise bei Temperaturen zwischen 0°C und 150°C.

Zur Durchführung des erfindungsgemäßen Verfahrens setzt man je Mol an Carbonsäurederivat der Formel (II) im allgemeinen 1 bis 5 Mol, vorzugsweise 1,0 bis 2,5 Mol an Amin ein.

Die Reaktionsdurchführung, Aufarbeitung und Isolierung der Reaktionsprodukte erfolgt nach bekannten Verfahren (vgl. Herstellungsbeispiel).

Das erfindungsgemäße Verfahren kann auch als zweistufiger Prozess durchgeführt werden. Dabei werden die Carbonsäurederivate der allgemeinen Formel (II) zunächst in eine aktivierte Form überführt und in einem anschließenden Schritt mit den Aminen der allgemeinen Formel (III) zu den erfindungsgemäßen Iminoessigsäurederivaten der allgemeinen Formel (I) umgesetzt.

Als aktivierte Form der Carbonsäurederivate der Formel (II) kommen alle Carboxy-aktivierten Derivate infrage, wie z.B. Säurehalogenide, bevorzugt Säurechloride, Säureazide, ferner symmetrische und gemischte Anhydride, wie beispielsweise die gemischten O-Alkylkohlensäureanhydride, weiterhin aktivierte Ester, wie z.B. p-Nitrophenylester oder N-Hydroxisuccinimidester sowie Addukte mit Kondensationsmitteln, wie z.B. Dicyclohexylcarbodiimid oder in situ erzeugte aktivierte Formen der Carbonsäuren.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis,
Plasmopara-Arten, wie beispielsweise Plasmopara viticola,
Bremia-Arten, wie beispielsweise Bremia lactucae,
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae,
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei werden die erfindungsgemäßen Wirkstoffe mit besonders gutem Erfolg zur Bekämpfung von Krankheiten im Wein-, Obst- und Gemüseanbau, wie beispielsweise gegen Plasmopara- und Phytophthora-Arten eingesetzt. Mit gutem Erfolg werden auch Reiskrankheiten, wie beispielsweise Pyriculariaarten bekampft.

Die Wirkstoffe werden in Abhängigkeit von ihren jeweiligen physikalischen und/oder chemischen Eigenschaften in die ublichen Formulierungen ubergeführt, wie Lösungen, Emulsionen, Suspensionen, Pulver, Schaume, Pasten, Granulate, Aerosole, Feinstverkapselungen in polymeren Stoffen und in Hüllmassen fur Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline; Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Caicit. Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexformige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe werden als solche oder in ihren Formulierungen auch in Mischung mit bekannten Fungiziden, Bakteriziden, Akariziden, Nematiziden oder Insektiziden verwendet, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

In vielen Fällen werden dabei synergistische Wirkungen beobachtet.

Besonders günstige Mischpartner sind z.B. die folgenden Verbindungen:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazole-5-carboxanilid; 2,6-Dichloro-N-(4-trifluoromethylbenzyl)benzamid; (E)-2-Methoxyimino-N-methyl-2-(2-phenoxyphenyl)-acetamid; 8-Hydroxychinolinsulfat: Methyl-(E)-2-{2-[6-(2-cyanophenoxy)pyrimidin-4-yloxy]-phenyl}-3-methoxyacrylat; Methyl-(E)-methoximino [alpha-(o-tolyloxy)-o-tolyl] acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid. Diclomezin, Dicloran, Diethofencarb. Difenoconazol, Dimethirimol, Dimethomorph, Diniconazol, Dinocap, Diphenylamin, Dipyrithion, Ditalimfos, Dithianon, Dodine, Drazoxolon, Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer and Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen

### Insektizide / Akarizide / Nematizide:

Abamectin, Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, Chloethocarb, Chlorethoxyfos, Chloretoxyfos, Chlorfenvinphos, Chlorfluazuron, Chlormephos, N-[(6-Chloro-3-pyridinyl)-methyl]-N'-cyano-N-methylethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb, HCH, Heptenophos, Hexaflumuron, Hexythiazox,
Imidacloprid, Iprobenfos, Isazophos, Isofenphos. Isoprocarb, Isoxathion, Ivemectin, Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, Pirimiphos A, Profenofos, Profenophos, Promecarb, Propaphos, Propoxur, Prothiofos, Prothiophos, Prothoat, Pymetrozin, Pyrachlophos, Pyraclofos, Pyraclophos, Pyradaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin. Tralomethrin, Triarathen, Triazophos, Triazuron, Trichlorfon, Triflumuron, Trimethacarb.
Vamidothion, XMC, Xylylcarb, YI 5301/5302, Zetamethrin.

Auch eine Mischung mit anderen bekannten Wirkstoffen, wie Herbiziden oder mit Düngemitteln und Wachstumsregulatoren ist möglich.

Die erfindungsgemäßen Wirkstoffe werden als solche, in Form ihren handelsüblichen Formulierungen oder den daraus bereiteten Anwendungsformen, wie gebrauchsfertige Lösungen, Suspensionen, Spritzpulver, Pasten, lösliche Pulver, Stäubemittel und Granulate angewendet. Die Anwendung geschieht in üblicher Weise, z.B. durch Gießen, Verspritzen, Versprühen, Verstreuen, Verschäumen, Bestreichen usw. Gegebenenfalls werden die Wirkstoffe nach dem Ultra-Low-Volume-Verfahren ausgebracht oder die Wirkstoffzubereitung oder der Wirkstoff selbst wird in den Boden injiziert. Gegebenenfalls wird auch das Saatgut der Pflanzen behandelt.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele:

### Beispiel 1:

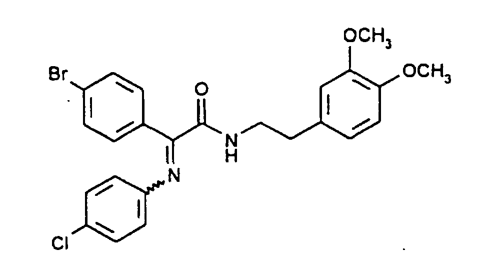

Es werden 5,5 g (0,015 Mol) 2-(4-Chlorphenylimino)-2-(4-bromphenyl)-essigsäureethylester, 2,72 g (0,015 Mol) 2-(3,4-Dimethoxyphenyl)-ethylamin und 5,4 g (0,03 Mol) 30%ige methanolische Natriummethylatlösung in 30 ml Methanol 18 Stunden zum Sieden erhitzt. Nach Abdestillieren des Lösungsmittels wird der Rückstand in Dichlormethan aufgenommen, nacheinander mit 1N-Salzsäure und Wasser gewaschen und die organische Phase über Natriumsulfat getrocknet. Das Lösungsmittel wird bei vermindertem Druck abdestilliert und der Rückstand mit Petrolether/Essigester (3:1) an Kieselgel chromatografiert. Man erhält 2,5 g (33 % der Theorie) eines Gemisches von e- und z-N-[2-(3.4-Dimethoxyphenyl)-ethyl]-2-(4-chlorphenylimino)-2-(4-bromphenyl)-essigsaureamid als Öl
¹H-NMR (CDCl₃. TMS) δ (ppm) = 3.87 (s, 3H)

Analog Beispiel 1, sowie entsprechend den Angaben in der allgemeinen Verfahrensbeschreibung, werden die in der nachstehenden Tabelle 6 genannten Verbindungen der Formel (le) erhalten.

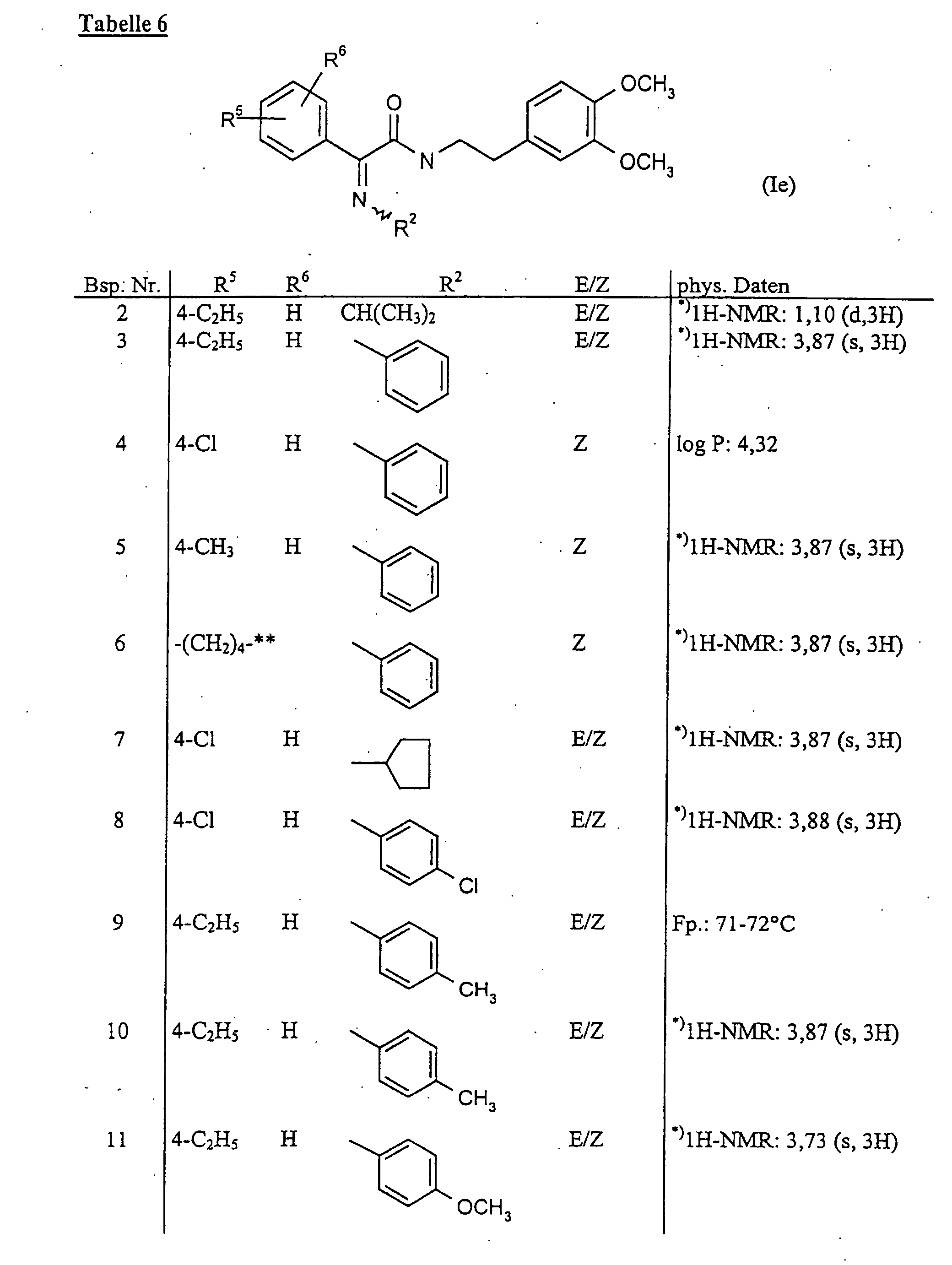

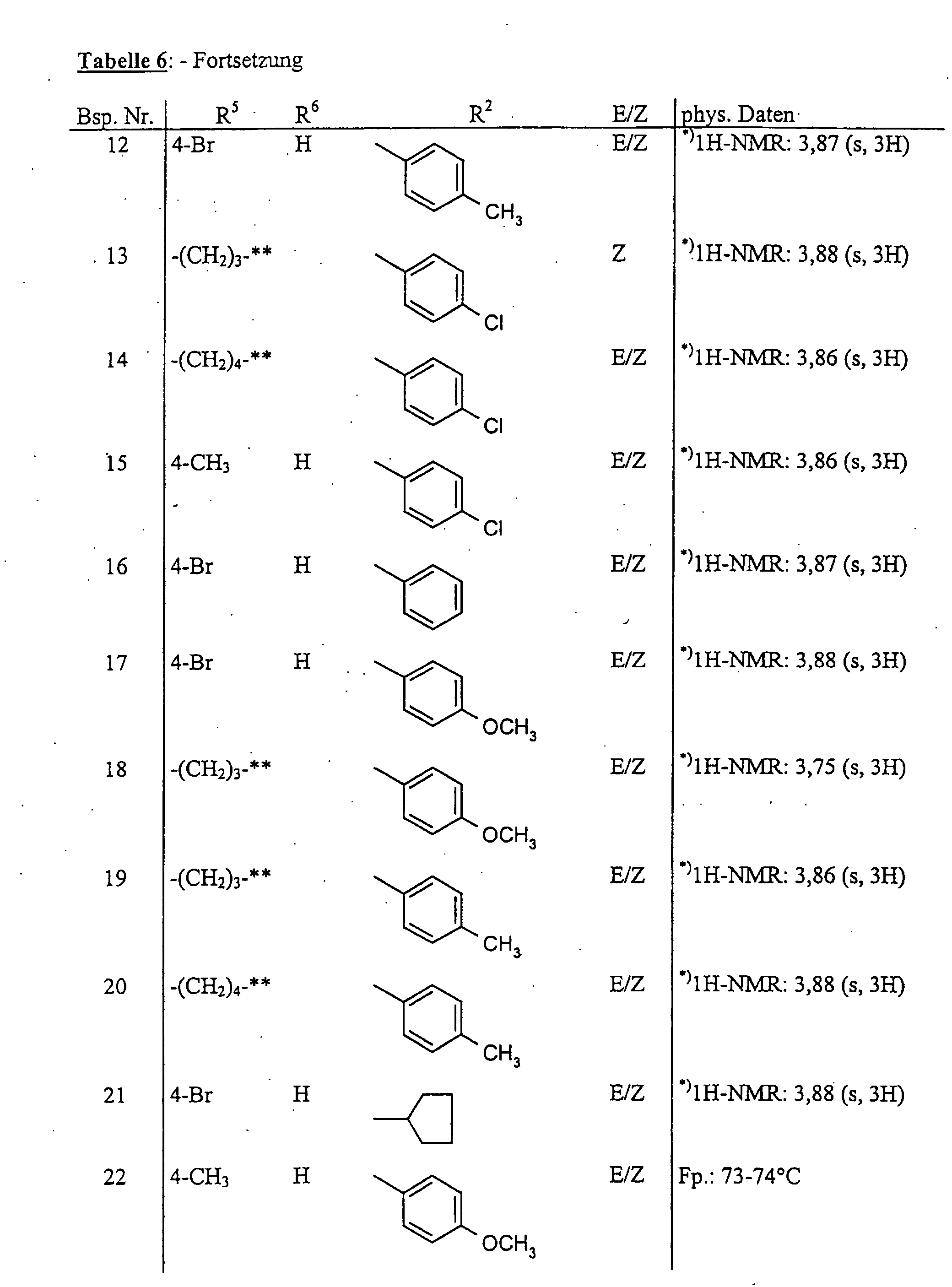

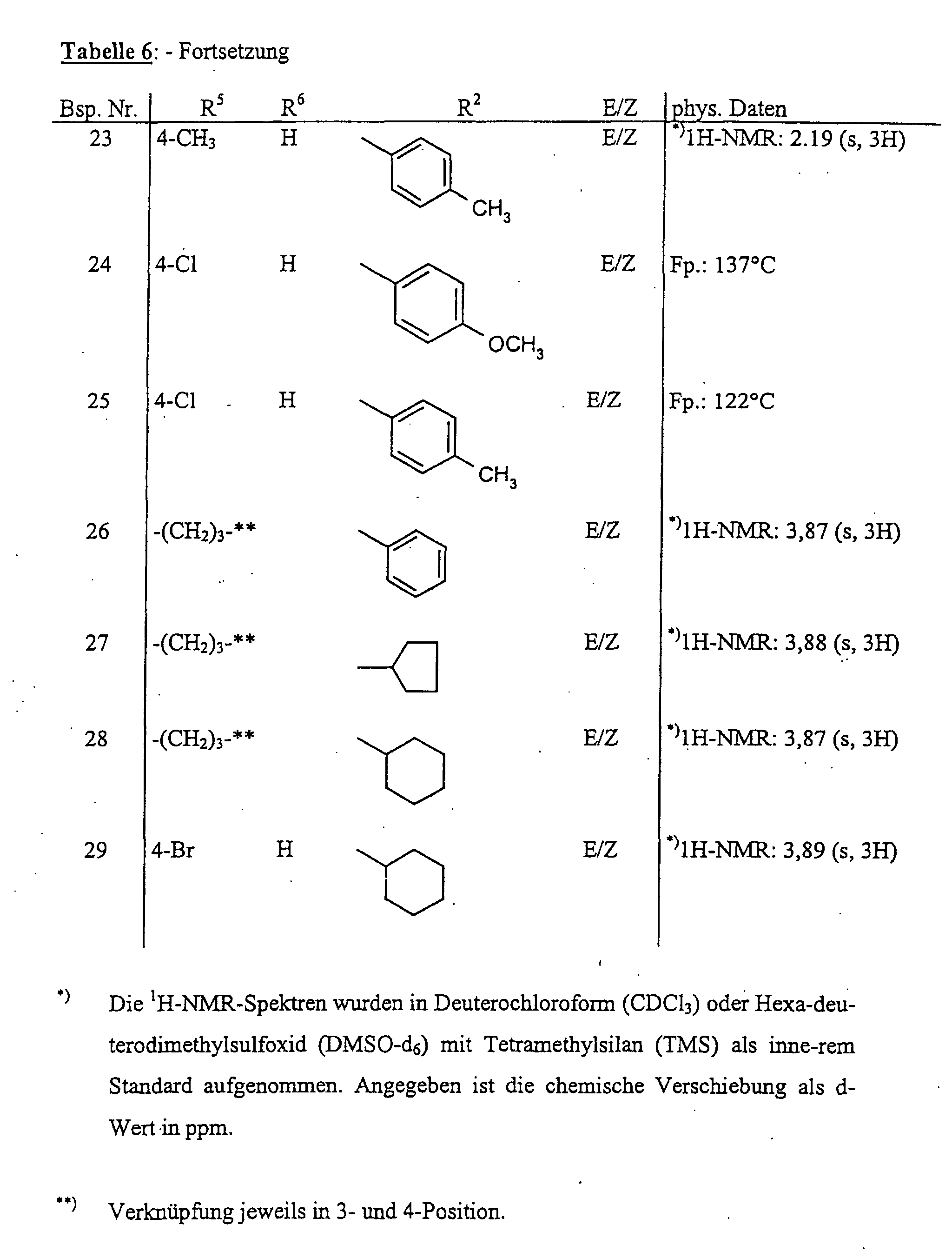

### Anwendungsbeispiele

### Beispiel A

### Phytophthora-Test (Tomate) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewunschte Konzentration.

Zur Prüfung auf protektive Wirksamkeit werden junge Pflanzen mit der Wirkstoffzubereitung besprüht. Nach Antrocknen des Spritzbelages werden die Pflanzen mit einer wäßrigen Sporensuspension von Phytophthora infestans inokuliert

Die Pflanzen werden in einer Inkubationskabine mit 100% relativer Luftfeuchtigkeit und ca 20°C aufgestellt.

3 Tage nach der Inokulation erfolgt die Auswertung

Bei diesem Test zeigen z.B. die folgenden Verbindungen (1), (6), (7), (16), (17), (18) und (24) der Herstellungsbeispiele bei einer Wirkstoffkonzentration von 50 ppm einen Wirkungsgrad von bis zu 98 %

## Patentansprüche

1. Verbindungen der Formel (I), in welcher 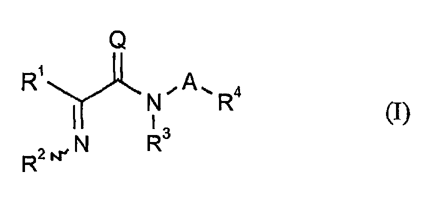
A für eine Einfachbindung oder für gegebenenfalls einfach oder mehrfach, gleich oder verschieden durch Fluor, Chlor, Cyano, Methoxy substituiertes Methylen, 1,2-Ethylen 1,1-, 1,2-, 1,3- oder 2,2-Propylen, 1,1-, 1,2-, 1,3-, 1,4-, 2,2- oder 2,3-Butylen oder 1,1-, 1,2- oder 1,3-(2-Methyl-propylen) steht,
Q für Sauerstoff oder Schwefel steht,
R¹ für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluomiethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy;
Cyclopropyl, Cyclobutyl, Cyclopentyl oder Cyclohexyl;
gegebenenfalls durch die oben genannten Substituenten substituiertes Phenyl, Benzyl, Phenoxy, Benzyloxy,
R² für Methyl, Ethyl, n- oder i-Propyl, n-, i-, s- oder t-Butyl oder Propargyl oder Allyl steht,
oder für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
oder für die oben genannten Substituenten substituiertes Benzyl steht,
R³ für Wasserstoff oder für Methyl oder Ethyl steht,
R⁴ für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

2. Verbindungen der Formel (I) gemäß Anspruch 1, in welcher
A für eine Einfachbindung oder für gegebenenfalls einfach oder mehrfach, gleich oder verschieden durch Fluor, Chlor, Cyano, Methoxy substituiertes Methylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
Q für Sauerstoff oder Schwefel steht,
R¹ für jeweils gegebenenfalls einfach bis sechsfach substituiertes Cyclobutyl, Cyclopentyl oder Cyclohexyl, wobei als Substituenten die unten genannten bevorzugt sind;
für jeweils gegebenenfalls einfach bis dreifach substituiertes Phenyl, Naphthyl, Furyl, Benzofuranyl, Thienyl, Benzothienyl, Pyridyl, Chinolyl, Tetrahydrofuryl oder Perhydropyranyl steht, wobei die möglichen Substituenten vorzugsweise aus der nachstehenden Aufzählung ausgewählt sind:
Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl; jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy; Cyclopropyl, Cyclopentyl, Cyclohexyl; gegebenenfalls durch die oben genannten Substituenten substituierter Phenyl, Phenoxy, Benzyloxy;
R² für Methyl, Ethyl, n-, i-Propyl, n-, i-, s- oder t-Butyl, Allyl
oder für jeweils gegebenenfalls einfach bis sechsfach substituiertes Cyclobutyl, Cyclopentyl oder Cyclohexyl, wobei als Substituenten die unten genannten bevorzugt sind;
oder für jeweils gegebenenfalls einfach bis dreifach substituiertes Phenyl, Naphthyl, Furyl, Benzofuranyl, Thienyl, Benzothienyl, Pyridyl, Chinolyl, Tetrahydrofuryl oder Perhydropyranyl steht, wobei die möglichen Substituenten vorzugsweise aus der nachstehenden Aufzählung ausgewählt sind:
Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl; jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
oder für die oben genannten Substituenten substituiertes Benzyl steht,
R³ für Wasserstoff oder für Methyl steht,
R⁴ für jeweils gegebenenfalls einfach bis sechsfach substituiertes Cyclopropyl, Cyclobutyl, Cyclopentyl oder Cyclohexyl steht, wobei als Substituenten die unten genannten bevorzugt sind;
für jeweils gegebenenfalls einfach bis dreifach substituiertes Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3diyl), Tetramethylen (Butan-1,4-diyl), Methylendioxy oder Ethylendioxy,
Cyclopropyl, Cyclobutyl, Cyclopentyl oder Cyclohexyl.

3. Verbindungen der Formel (I) gemäß Anspruch 1, in welcher
A für eine Einfachbindung oder für Methylen, 1,2-Ethylen, 1,1-Propylen, 1,2-Propylen, 1,3-Propylen oder 2,2-Propylen steht,
Q für Sauerstoff steht,
R¹ für gegebenenfalls durch Brom, Chlor, Fluor, Nitro, Methylsulfonyl, Phenyl, Phenyloxy, Benzyloxy, Cyclopropyl, Cyclohexyl, Methyl, Ethyl, n- oder i-Propyl, n-, i-, s- oder t-Butyl, n-Pentyl, n-Hexyl, n-Heptyl, Methoxy, Ethoxy, Trifluormethyl, Trifluormethoxy und/oder Methylthio einfach oder zweifach substituiertes Phenyl, Thienyl oder Furanyl steht oder für jeweils gegebenenfalls durch Fluor substituiertes 3,4-Methylen- und Ethylendioxo, Propan-1,3-diyl und Butan-1,4-diyl substituiertes Phenyl steht oder für Naphthyl, Benzofuranyl oder Benzothienyl steht,
R² für Methyl oder Ethyl, für n- oder i-Propyl, für n-, i-, s- oder t-Butyl, Allyl, Cyclopentyl, Cyclohexyl oder für gegebenenfalls durch Chlor, Methyl oder Methoxy substituiertes Phenyl oder Benzyl steht,
R³ für Wasserstoff oder Methyl steht,
R⁴ für Cyclohexyl oder gegebenenfalls einfach bis dreifach substituiertes Phenyl, Thienyl, Furyl, Benzofuryl, Benzothienyl, Pyridyl, Pyrimidinyl, Naphthyl, Chinolyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy steht.

4. Fungizide Mittel, **gekennzeichnet durch** einen Gehalt an mindestens einer Verbindung der Formel (I) nach Anspruch 1 mit Streckmitteln und/oder oberflächenaktiven Mitteln vermischt.

5. Verfahren zur Bekämpfung von Schädlingen, **dadurch gekennzeichnet, dass** man Verbindungen der Formel (I) nach Anspruch 1 auf Schädlinge und/oder ihren Lebensraum einwirken läßt.

6. Verwendung von Verbindungen bzw. Mitteln der Formel (I) nach den Ansprüchen 1 bis 3 zur Bekämpfung von Schädlingen.

7. Verfahren zur Herstellung von Schädlingsbekämpfungsmitteln, **dadurch gekennzeichnet, dass** man Verbindungen der Formel (I) nach den Ansprüchen 1 bis 3 mit Streckmitteln und/oder oberflächenaktiven Mitteln vermischt.

8. Verfahren zur Herstellung von Verbindungen der Formel (I) nach Anspruch 1, **dadurch gekennzeichnet, daß** man Carbonsäurederivate der allgemeinen Formel (II) 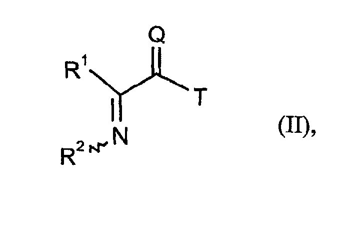 in welcher
R¹, R² und Q die in Anspruc h 1 angegebenen Bedeutungen haben und
T für Hydroxy, Halogen oder Alkoxy steht,
mit einem Amin der allgemeinen Formel (III), 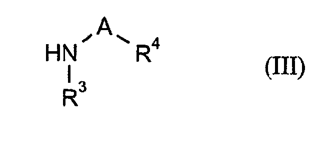 in welcher
R³, R⁴ und A die in Anspruch 1 angegebenen Bedeutungen haben,
- oder mit einem Hydrogenhalogenid hiervon -
gegebenenfalls in Gegenwart eines Säureakzeptors, gegebenenfalls in Gegenwart eines Kondensationsmittels, gegebenenfalls in Gegenwart eines Katalysators und gegebenenfalls in Gegenwart eines Verdünnungsmittels umsetzt.

## Claims

1. Compounds of the formula (I) 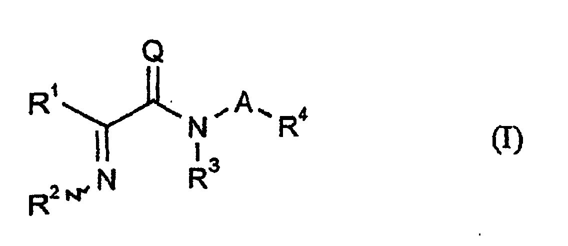 in which
A represents a single bond or represents methylene, 1,2-ethylene 1,1-, 1,2-, 1,3- or 2,2-propylene, 1,1-, 1,2-, 1,3-, 1,4-, 2,2- or 2,3-butylene or 1,1-, 1,2- or 1,3-(2-methylpropylene), each of which is optionally mono- or polysubstituted by identical or different substituents from the group consisting of fluorine, chlorine, cyano and methoxy,
Q represents oxygen or sulphur,
R¹ represents respectively optionally mono- to trisubstituted cyclopropyl, cyclobutyl, cyclopentyl or cyclohexyl, phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, the possible substituents preferably being selected from the list below:
fluorine, chlorine, bromine, cyano, nitro, amino, hydroxyl, formyl, carboxy, carbamoyl, thiocarbamoyl, methyl, ethyl, n- or i-propyl, n-, i-, s- or t-butyl, n-pentyl, n-hexyl, n-heptyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroxyiminomethyl, hydroxyiminoethyl, methoxyiminomethyl, ethoxyiminomethyl, methoxyiminoethyl or ethoxyiminoethyl;
respectively doubly attached trimethylene (propane-1,3-diyl), tetramethylene (butane-1,4-diyl), methylenedioxy or ethylenedioxy, each of which is optionally mono- or polysubstituted by identical or different substituents from the group consisting of fluorine, chlorine, methyl, trifluoromethyl, ethyl, n- and i-propyl;
cyclopropyl, cyclobutyl, cyclopentyl or cyclohexyl;
phenyl, benzyl, phenoxy, benzyloxy, each of which is optionally substituted by the abovementioned substituents;
R² represents methyl, ethyl, n- or i-propyl, n-, i-, s- or t-butyl or propargyl or allyl,
or represents respectively optionally mono- to trisubstituted cyclopropyl, cyclobutyl, cyclopentyl or cyclohexyl, phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, the possible substituents preferably being selected from the list below:
fluorine, chlorine, bromine, cyano, nitro, amino, hydroxyl, formyl, carboxy, carbamoyl, thiocarbamoyl, methyl, ethyl, n- or i-propyl, n-, i-, s- or t-butyl, n-pentyl, n-hexyl, n-heptyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroxyiminomethyl, hydroxyiminoethyl, methoxyiminomethyl, ethoxyiminomethyl, methoxyiminoethyl or ethoxyiminoethyl;
respectively doubly attached trimethylene (propane-1,3-diyl), tetramethylene (butane-1,4-diyl), methylenedioxy or ethylenedioxy, each of which is optionally mono- or polysubstituted by identical or different substituents from the group consisting of fluorine, chlorine, methyl, trifluoromethyl, ethyl, n- and i-propyl;
or represents benzyl which is substituted the abovementioned substituents,
R³ represents hydrogen or represents methyl or ethyl,
R⁴ represents optionally mono- to trisubstituted cyclopropyl, cyclobutyl, cyclopentyl or cyclohexyl, phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, the possible substituents preferably being selected from the list below:
fluorine, chlorine, bromine, cyano, nitro, amino, hydroxyl, formyl, carboxy, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroxyiminomethyl, hydroxyiminoethyl, methoxyiminomethyl, ethoxyiminomethyl, methoxyiminoethyl or ethoxyiminoethyl;
respectively doubly attached trimethylene (propane-1,3-diyl), tetramethylene (butane-1,4-diyl), methylenedioxy or ethylenedioxy, each of which is optionally mono- or polysubstituted by identical or different substituents from the group consisting of fluorine, chlorine, methyl, trifluoromethyl, ethyl, n- and i-propyl,
cyclopropyl, cyclobutyl, cyclopentyl or cyclohexyl.

2. Compounds of the formula (I) according to Claim 1, in which
A represents a single bond or represents methylene, 1,2-ethylene 1,1-, 1,2-, 1,3- or 2,2-propylene, 1,1-, 1,2-, 1,3-, 1,4-, 2,2-, 2,3-butylene or 1,1-, 1,2- or 1,3-(2-methyl-propylene), each of which is optionally mono- or polysubstituted by identical or different substituents from the group consisting of fluorine, chlorine, cyano and methoxy,
Q represents oxygen or sulphur,
R¹ represents respectively optionally mono- to hexasubstituted cyclobutyl, cyclopentyl or cyclohexyl, preferred substituents being those listed below;
represents respectively optionally mono- to trisubstituted phenyl, naphthyl, furyl, benzofuranyl, thienyl, benzothienyl, pyridyl, quinolyl, tetrahydrofuryl or perhydropyranyl, the possible substituents preferably being selected from the list below:
fluorine, chlorine, bromine, cyano, nitro, methyl, ethyl, n- or i-propyl, n-, i, s- or t-butyl, n-pentyl, n-hexyl, n-heptyl, methoxy, ethoxy, n- or i- propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl, trifluoromethylsulphonyl, acetyl, propionyl, acetyloxy, methoxycarbonyl, ethoxycarbonyl, methylsulphonyloxy, ethylsulphonyloxy, hydroxyiminomethyl, hydroxyiminoethyl, methoxyiminomethyl, ethoxyiminomethyl, methoxyiminoethyl or ethoxyiminoethyl;
respectively doubly attached trimethylene (propane-1,3-diyl), tetramethylene (butane-1,4-diyl), methylenedioxy or ethylenedioxy, each of which is optionally mono- or polysubstituted by identical or different substituents from the group consisting of fluorine, chlorine, methyl, trifluoromethyl, ethyl, n- and i-propyl; cyclopropyl, cyclopentyl, cyclohexyl; phenyl, phenoxy, benzyloxy, each of which is optionally substituted by the abovementioned substituents;
R² represents methyl, ethyl, n-, i-propyl, n-, i-, s- or t-butyl, allyl
or represents respectively optionally mono- to hexasubstituted cyclobutyl, cyclopentyl or cyclohexyl, preferred substituents being those listed below;
or represents respectively optionally mono- to trisubstituted phenyl, naphthyl, furyl, benzofuranyl, thienyl, benzothienyl, pyridyl, quinolyl, tetrahydrofuryl or perhydropyranyl, the possible substituents preferably being selected from the list below:
fluorine, chlorine, bromine, cyano, nitro, methyl, ethyl, n- or i-propyl, n-, i, s- or t-butyl, n-pentyl, n-hexyl, n-heptyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl, trifluoromethylsulphonyl, acetyl, propionyl, acetyloxy, methoxycarbonyl, ethoxycarbonyl, methylsulphonyloxy, ethylsulphonyloxy, hydroxyiminomethyl, hydroxyiminoethyl, methoxyiminomethyl, ethoxyiminomethyl, methoxyiminoethyl or ethoxyiminoethyl;
respectively doubly attached trimethylene (propane-1,3-diyl), tetramethylene (butane-1,4-diyl), methylenedioxy or ethylenedioxy, each of which is optionally mono- or polysubstituted by identical or different substituents from the group consisting of fluorine, chlorine, methyl, trifluoromethyl, ethyl, n- or i-propyl,
or represents benzyl which is substituted the abovementioned substituents,
R³ represents hydrogen or represents methyl,
R⁴ represents respectively optionally mono- to hexasubstituted cyclopropyl, cyclobutyl, cyclopentyl or cyclohexyl, preferred substituents being those listed below;
represents respectively optionally mono- to trisubstituted phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, the possible substituents preferably being selected from the list below:
fluorine, chlorine, bromine, cyano, nitro, amino, hydroxyl, formyl, carboxy, carbamoyl, thiocarbamoyl, methyl, ethyl, n- or i-propyl, n-, i-, s- or t-butyl, methoxy, ethoxy, n- or i-propoxy, methylthio, ethylthio, n- or i- propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroxyiminomethyl, hydroxyiminoethyl, methoxyiminomethyl, ethoxyiminomethyl, methoxyiminoethyl or ethoxyiminoethyl; respectively doubly attached trimethylene (propane-1,3-diyl), tetramethylene (butane-1,4-diyl), methylenedioxy or ethylenedioxy, each of which is optionally mono- or polysubstituted by identical or different substituents from the group consisting of fluorine, chlorine, methyl, trifluoromethyl, ethyl, n- and i-propyl;
cyclopropyl, cyclobutyl, cyclopentyl or cyclohexyl.

3. Compounds of the formula (I) according to Claim 1, in which
A represents a single bond or represents methylene, 1,2-ethylene, 1,1-propylene, 1,2-propylene, 1,3-propylene or 2,2-propylene,
Q represents oxygen,
R¹ represents phenyl, thienyl or furanyl, each of which is optionally mono- or disubstituted by bromine, chlorine, fluorine, nitro, methylsulphonyl, phenyl, phenyloxy, benzyloxy, cyclopropyl, cyclohexyl, methyl, ethyl, n- or i-propyl, n-, i-, s- or t-butyl, n-pentyl, n-hexyl, n-heptyl, methoxy, ethoxy, trifluoromethyl, trifluoromethoxy and/or methylthio, or represents phenyl which is substituted by 3,4-methylene- and ethylenedioxo, propane-1,3-diyl and butane-1,4-diyl, each of which is optionally substituted by fluorine, or represents naphthyl, benzofuranyl or benzothienyl,
R² represents methyl or ethyl, represents n- or i-propyl, represents n-, i-, s- or t-butyl, allyl, cyclopentyl, cyclohexyl or represents optionally chlorine-, methyl- or methoxy-substituted phenyl or benzyl,
R³ represents hydrogen or methyl,
R⁴ represents cyclohexyl or optionally mono- to trisubstituted phenyl, thienyl, furyl, benzofuryl, benzothienyl, pyridyl, pyrimidinyl, naphthyl, quinolyl, the possible substituents preferably being selected from the list below:
fluorine, chlorine, bromine, cyano, nitro, amino, hydroxyl, formyl, carboxy, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroxyiminomethyl, hydroxyiminoethyl, methoxyiminomethyl, ethoxyiminomethyl, methoxyiminoethyl or ethoxyiminoethyl,
respectively doubly attached trimethylene (propane-1,3-diyl), tetramethylene (butane-1,4-diyl), methylenedioxy or ethylenedioxy, each of which is optionally mono- or polysubstituted by identical or different substituents from the group consisting of fluorine, chlorine, methyl, trifluoromethyl, ethyl, n- and i-propyl.

4. Fungicides, **characterized in that** they comprise at least one compound of formula (I) according to Claim 1 mixed with extenders and/or surfactants.

5. Method for controlling pests, **characterized in that** compounds of the formula (I) according to Claim I are allowed to act on pests and/or their habitat.

6. Use of compounds or compositions of the formula (I) according to Claims I to 3, for controlling pests.

7. Process for preparing pesticides, **characterized in that** compounds of the formula (I) according to Claims 1 to 3 are mixed with extenders and/or surfactants.

8. Process for preparing compounds of the formula (I) according to Claim 1, **characterized in that** carboxylic acid derivatives of the general formula (II) 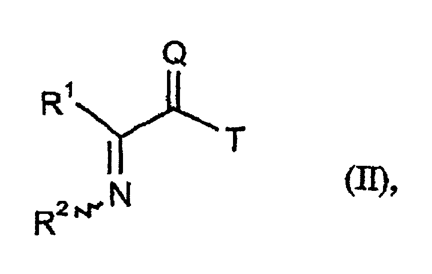 in which
R¹, R² and Q are each as defined in Claim 1 and
T represents hydroxyl, halogen or alkoxy,
are reacted with an amine of the general formula (III), 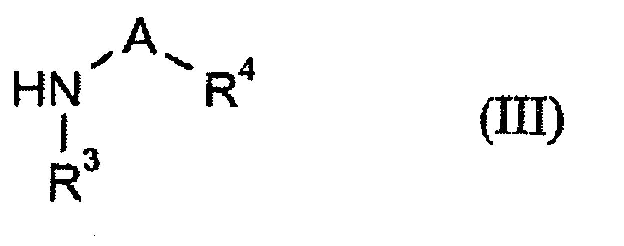 in which
R³, R⁴ and A are each as defined in Claim 1,
- or with a hydrogen halide thereof -
if appropriate in the presence of an acid acceptor, if appropriate in the presence of a condensing agent, if appropriate in the presence of a catalyst and if appropriate in the presence of a diluent.

## Revendications

1. Composés de formule (I) 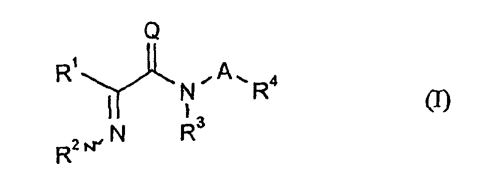 dans laquelle
A représente une liaison simple ou un groupe méthylène portant éventuellement un ou plusieurs substituants fluoro, chloro, cyano, méthoxy identiques ou différents, un groupe 1,2-éthylène, 1,1-, 1,2-, 1,3- ou 2,2-propylène, 1,1-, 1,2-, 1,3-, 1,4-, 2,2- ou 2,3-butylène ou 1,1-, 1,2- ou 1,3-(2-méthylpropylène),
Q représente l'oxygène ou le soufre,
R¹ est un reste cyclopropyle, cyclobutyle, cyclopentyle, cyclohexyle, phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle dont chacun porte éventuellement un à trois substituants, les substituants possibles étant choisis de préférence parmi ceux qui sont énumérés ci-après : fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertio-butyle, n-pentyle, n-hexyle, n-heptyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximino-éthyle, méthoximinométhyle, éthoximinométhyle, méthoximino-éthyle ou éthoximino-éthyle ; un reste triméthylène (propane-1,3-diyle), tétraméthylène (butane-1,4-diyle), méthylènedioxy ou éthylènedioxy ayant chacun deux liaisons et portant chacun, le cas échéant, un ou plusieurs substituants, identiques ou différents, fluoro, chloro, méthyle, trifluorométhyle, éthyle, n-propyle ou isopropyle ;
un reste cyclopropyle, cyclobutyle, cyclopentyle ou cyclohexyle ;
un reste phényle portant éventuellement les substituants mentionnés ci-dessus, un reste benzyle, phénoxy ou benzyloxy,
R² est un reste méthyle, éthyle, n-propyle, isopropyle, n-butyle, isobutyle, sec.-butyle, tertio-butyle, propargyle ou allyle,
ou bien un reste cyclopropyle, cyclobutyle, cyclopentyle, cyclohexyle, phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle, dont chacun porte éventuellement un à trois substituants, les substituants possibles étant avantageusement choisis parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertio-butyle, n-pentyle, n-hexyle, n-heptyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximino-éthyle, méthoximinométhyle, éthoximinométhyle, méthoximino-éthyle ou éthoximino-éthyle ;
un reste triméthylène (propane-1,3-diyle), tétraméthylène (butane-1,4-diyle), méthylènedioxy ou éthylènedioxy ayant chacun deux liaisons et portant chacun, le cas échéant, un ou plusieurs substituants fluoro, chloro, méthyle, trifluorométhyle, éthyle, n- propyle ou isopropyle identiques ou différents,
ou bien un reste benzyle substitué avec les substituants mentionnés ci-dessus,
R³ représente l'hydrogène ou un reste méthyle ou éthyle,
R⁴ est un reste cyclopropyle, cyclobutyle, cyclopentyle, cyclohexyle, phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle dont chacun porte, le cas échéant, un à trois substituants, les substituants possibles étant avantageusement choisis parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximino-éthyle, méthoximinométhyle, éthoximinométhyle, méthoximino-éthyle ou éthoximino-éthyle,
un reste triméthylène (propane-1,3-diyle), tétraméthylène (butane-1,4-diyle), méthylènedioxy ou éthylènedioxy ayant chacun deux liaisons et portant chacun, le cas échéant, un ou plusieurs substituants, fluoro, chloro, méthyle, trifluorométhyle, éthyle, n-propyle ou isopropyle identiques ou différents, un reste cyclopropyle, cyclobutyle, cyclopentyle ou cyclohexyle.

2. Composés de formule (I) suivant la revendication 1, formule dans laquelle :
A représente une liaison simple ou un reste méthylène éventuellement substitué une ou plusieurs fois identiques ou différentes par un radical fluoro, chloro, cyano, méthoxy, un reste 1,2-éthylène, 1,1-, 1,2-, 1,3- ou 2,2-propylène, 1,1-, 1,2-, 1,3-, 1,4-, 2,2-, 2,3-butylène ou 1,1-, 1,2- ou 1,3-(2-méthylpropylène),
Q représente l'oxygène ou le soufre,
R¹ est un reste cyclobutyle, cyclopentyle ou cyclohexyle portant chacun, le cas échéant, un à six substituants, avec comme substituants appréciés ceux qui sont mentionnés ci-dessous :
un reste phényle, naphtyle, furyle, benzofurannyle, thiényle, benzothiényle, pyridyle, quinolyle, tétrahydrofuryle ou perhydropyrannyle portant chacun, le cas échéant, un à trois substituants, les substituants possibles étant avantageusement choisis parmi ceux qui sont énumérés ci-après :
fluor, chlore, brome, cyano, nitro, méthyle, éthyle, n-propyle, isopropyle, n-butyle, isobutyle, sec.-butyle, tertio-butyle, n-pentyle, n-hexyle, n-heptyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, acétyle, propionyle, acétyloxy, méthoxycarbonyle, éthoxycarbonyle, méthylsulfonyloxy, éthylsulfonyloxy, hydroximinométhyle, hydroximino-éthyle, méthoximinométhyle, éthoximinométhyle, méthoximino-éthyle ou éthoximino-éthyle ;
un reste triméthylène (propane-1,3-diyle), tétraméthylène (butane-1,4-diyle), méthylènedioxy ou éthylènedioxy ayant chacun deux liaisons et portant chacun, le cas échéant, un ou plusieurs substituants fluoro, chloro, méthyle, trifluorométhyle, éthyle, n-propyle ou isopropyle identiques ou différents;
un reste cyclopropyle, cyclopentyle, cyclohexyle ;
un reste phényle, phénoxy, benzyloxy portant éventuellement les substituants mentionnés ci-dessus ;
R² est un reste méthyle, éthyle, n-propyle, isopropyle, n-butyle, isobutyle, sec.-butyle, tertio-butyle, allyle,
ou bien un reste cyclobutyle, cyclopentyle, ou cyclohexyle portant chacun, le cas échéant, un à six substituants, avec comme substituants les substituants appréciés mentionnés ci-dessous ;
ou bien un reste phényle, naphtyle, furyle, benzofurannyle, thiényle, benzothiényle, pyridyle, quinolyle, tétrahydrofuryle ou perhydropyrannyle portant chacun, le cas échéant, un à trois substituants, les substituants possibles étant avantageusement choisis parmi ceux qui sont énumérés ci-après :
fluor, chlore, brome, cyano, nitro, méthyle, éthyle, n-propyle, isopropyle, n-butyle, isobutyle, sec.-butyle, tertio-butyle, n-pentyle, n-hexyle, n-heptyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, acétyle, propionyle, acétyloxy, méthoxycarbonyle, éthoxycarbonyle, méthylsulfonyloxy, éthylsulfonyloxy, hydroximinométhyle, hydroximino-éthyle, méthoximinométhyle, éthoximinométhyle, méthoximino-éthyle ou éthoximino-éthyle ;
un reste triméthylène (propane-1,3-diyle), tétraméthylène (butane-1,4-diyle), méthylènedioxy ou éthylènedioxy ayant chacun deux liaisons et portant chacun, le cas échéant, un ou plusieurs substituants fluoro, chloro, méthyle, trifluorométhyle, éthyle, n-propyle ou isopropyle identiques ou différents,
ou bien un reste benzyle substitué avec les substituants mentionnés ci-dessus,
R³ représente l'hydrogène ou le reste méthyle,
R⁴ est un reste cyclopropyle, cyclobutyle, cyclopentyle ou cyclohexyle portant chacun, le cas échéant, un à six substituants, avec comme substituants les substituants appréciés mentionnés ci-dessous ; un reste phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, 5 pyrrolidinyle, pipéridinyle ou morpholinyle portant chacun, le cas échéant, un à trois substituants, les substituants possibles étant avantageusement choisis parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propyl-amino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximino-éthyle, méthoximinométhyle, éthoximinométhyle, méthoximino-éthyle ou éthoximino-éthyle,
un reste triméthylène (propane-1,3-diyle), tétraméthylène (butane-1,4-diyle), méthylènedioxy ou éthylènedioxy ayant chacun deux liaisons et portant chacun, le cas échéant, un ou plusieurs substituants fluoro, chloro, méthyle, trifluorométhyle, éthyle, n-propyle ou isopropyle identiques ou différents, un reste cyclopropyle, cyclobutyle, cyclopentyle ou cyclohexyle.

3. Composés de formule (I) suivant la revendication 1, formule dans laquelle
A représente une liaison simple ou un reste méthylène, 1,2-éthylène, 1,1-propylène, 1,2-propylène, 1,3-propylène ou 2,2-propylène,
Q représente l'oxygène,
R¹ est un reste phényle portant éventuellement un ou deux substituants bromo, chloro, fluoro, nitro, méthylsulfonyle, phényle, phényloxy, benzyloxy, cyclopropyle, cyclohexyle, méthyle, éthyle, n-propyle, isopropyle, n-butyle, isobutyle, sec.-butyle, tertiobutyle, n-pentyle, n-hexyle, n-heptyle, méthoxy, éthoxy, trifluorométhyle, trifluorométhoxy et/ou méthylthio, un reste thiényle ou furannyle ou un reste phényle subtitué par un radical 3,4-méthylène- et éthylènedioxo, propane-1,3-diyle et butane-1,4-diyle dont chacun est éventuellement substitué par du fluor,
ou un reste naphtyle, benzofurannyle ou benzothiényle,
R² est un reste méthyle ou éthyle, un reste n-propyle ou isopropyle, un reste n-butyle, isobutyle, sec.-butyle
ou tertio-butyle, un reste allyle, cyclopentyle, cyclohexyle ou un reste phényle ou benzyle éventuellement substitué par du chlore, un radical méthyle ou méthoxy,
R³ représente l'hydrogène ou un reste méthyle,
R⁴ est un reste cyclohexyle ou un reste phényle, thiényle, furyle, benzofuryle, benzothiényle, pyridyle, pyrimidinyle, naphtyle, quinolyle portant éventuellement un à trois substituants, les substituants possibles étant choisis avantageusement parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximino-éthyle, méthoximinométhyle, éthoximinométhyle, méthoximino-éthyle ou éthoximino-éthyle,
un reste triméthylène (propane-1,3-diyle), tétraméthylène (butane-1,4-diyle), méthylènedioxy ou éthylènedioxy ayant chacun deux liaisons et dont chacun porte éventuellement un ou plusieurs substituants fluoro, chloro, méthyle, trifluorométhyle, éthyle, n-propyle ou isopropyle identiques ou différents.

4. Composition fongicide, **caractérisée par** une teneur en au moins un composé de formule (I) suivant la revendication 1, en mélange avec des diluants et/ou des agents tensio-actifs.

5. Procédé pour combattre des parasites, **caractérisé en ce qu'**on fait agir des composés de formule (I) suivant la revendication 1 sur les parasites et/ou sur leur milieu.

6. Utilisation de composés ou d'agents de formule (I) suivant les revendications 1 à 3 pour combattre des parasites.

7. Procédé de préparation de compositions pesticides, **caractérisé en ce qu'**on mélange des composés de formule (I) suivant les revendications 1 à 3 avec des diluants et/ou des agents tensio-actifs.

8. Procédé de production de composés de formule (I) suivant la revendication 1, **caractérisé en ce qu'**on fait réagir des dérivés d'acides carboxyliques de formule générale (II) 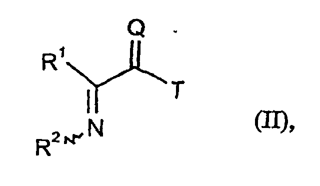 dans laquelle
R¹, R² et Q ont les définitions indiquées dans la revendication 1 et
T est un groupe hydroxy, un halogène ou un groupe alkoxy,
avec une amine de formule générale (III) 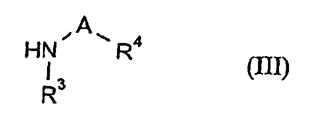 dans laquelle
R³, R⁴ et A ont les définitions indiquées dans la revendication 1,
- ou avec un halogénhydrate de cette amine -
le cas échéant en présence d'un accepteur d'acide, éventuellement en présence d'un agent de condensation, en présence éventuelle d'un catalyseur et, le cas échéant en présence d'un diluant.
